# EUROPEAN PATENT APPLICATION

(11) **EP 2 156 808 A2**
(43) Date of publication of application: **24.02.2010**
(21) Application number: 09167895.3
(22) Date of filing: 14.08.2009
(51) Int. Cl.: A61B 19/02

(54) **Medical Waste Container Lid**

(30) Priority: 14.08.2008 US 88871 P
(71) Applicant: Rehrig Pacific Company, Los Angeles, CA 90058 (US)
(72) Inventor: Apps, Willaim P, Alpharetta, Georgia 30022 (US)
(74) Representative: Lamb, Richard Andrew

(57) **Abstract**

The present invention provides a lid (32) for a waste container (10) having a first portion (32a) pivotably connected to a second portion (32b). The first portion (32a) includes a first handle (44a) and a first protrusion (58a) and the second portion (32b) includes a second handle (44b) and a second protrusion (58b). The first protrusion (58a) engages the second protrusion (58b) to prevent the first portion (32a) from rotating relative to the second portion (32b) past a threshold angle.

## Description

This disclosure generally relates to a lid for a medical waste disposal container. More particularly, this disclosure relates to a lid for accessing only a portion the waste container or the entire waste container.

Many types of containers have been used to store medical waste and protect the patient or health care professional from the waste product stored inside the container. The waste product may include biological material, medical devices, or other similar materials. It is important for the user disposing of the medical waste product to easily access the interior of the container to dispose of the medical waste.

It is desirable to provide an improved container and lid for such a container which addresses the above considerations and/or which more generally offers improvements or an alternative to existing arrangements.

According to the present invention there is therefore provided a lid for a waste container, and a method of removing a lid from a container, as described in the accompanying claims. There is also provided a container and lid assembly as further described in the accompanying claims.

In an embodiment of the invention there is provided a lid for a waste container having a first portion pivotably connected to a second portion. The first portion includes a first handle and a first protrusion and the second portion includes a second handle and a second protrusion. The first protrusion engages the second protrusion to prevent the first portion from rotating relative to the second portion past a threshold angle.

In a particular example shown, a lid and container assembly includes a lid and a container having an interior and an upper perimeter. The lid includes a first portion having a first protrusion, a second portion having a second protrusion, and a channel formed around a perimeter of the lid for accepting the upper perimeter of the container.

The lid is removed from the container by engaging a first handle on a first portion and a second handle on a second portion of the lid and applying a lifting force to the first handle and the second handle. A first protrusion on the first portion engages a second protrusion on the second portion of the lid to prevent the first portion from rotating relative to the second portion past a threshold angle.

The various features and advantages of the disclosed examples will become apparent to those skilled in the art from the following detailed description. The drawings that accompany the detailed description can be briefly described as follows:
Figure 1 illustrates an example container and lid;
Figure 2 illustrates a medical waste bag located on the container of Figure 1;
Figure 3 illustrates an example first portion of the lid of Figure 1;
Figure 4 illustrates a top perspective view of the lid of Figure 1;
Figure 5 illustrates a bottom perspective view of the lid of Figure 1;
Figure 6 illustrates a perspective view of a first portion of the lid of Figure 1 in an open position; and
Figure 7 illustrates a perspective view of a second portion of the lid of Figure 1 in an open position.

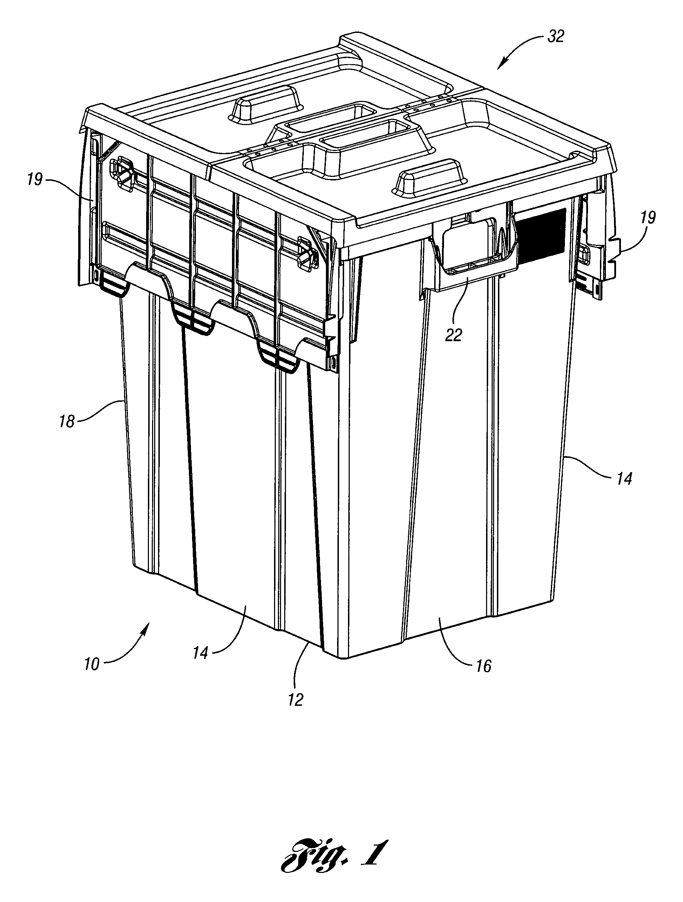

An example container 10 and lid 32 for collecting medical waste is shown in Figure 1. The medical waste could include biological material, medical devices, or other medical waste materials. Additionally, the container 10 could store recyclable medical devices, such as devices containing Titanium or other recyclable materials.

The container 10 includes a base wall 12, side walls 14, a front wall 16, a rear wall 18, and a pair of container flaps 19 hingeably connected to upper edges of the side walls 14. Handles 22 protrude outwardly from the front wall 16 and the rear wall 18.

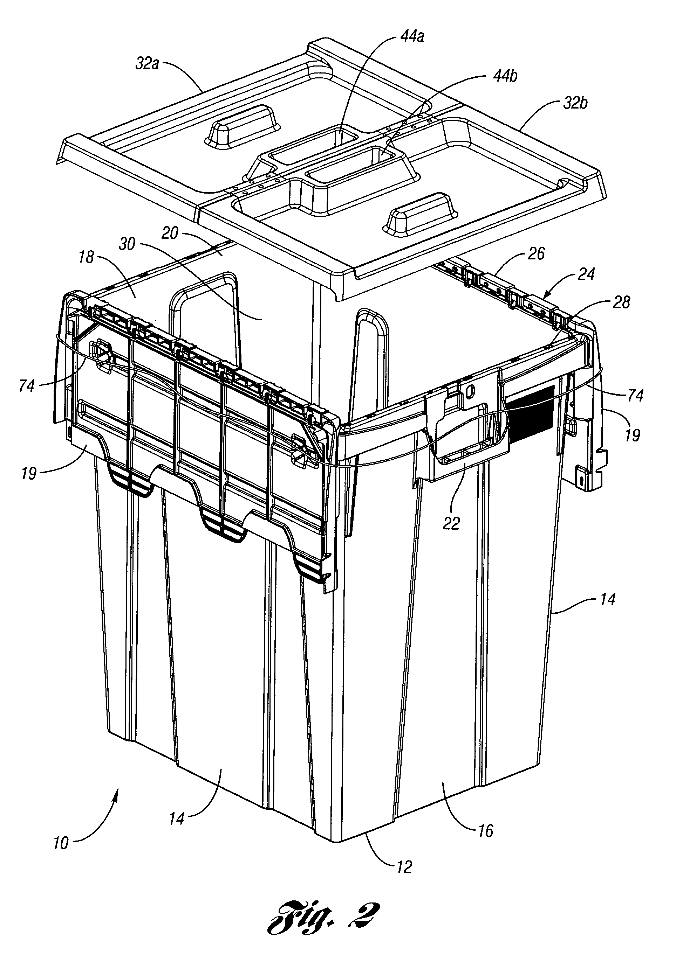

Figure 2 illustrates a container 10 lined with a medical waste bag 74. The medical waste bag 74 may drape over an upper edge 24 of the container 10. The upper edge 24 of the container 10 extends around an opening 30 and includes an upper hinged portion 26 and a lower flat portion 28. The upper hinged portion 26 connects to the container flaps 19 to provide a hinge connection between the side walls 14 and the container flaps 19. The upper hinged portion 26 is higher (i.e. spaced further from the base wall 12) than the lower flat portions 28 at the upper edges of the front and rear walls 16, 18. The lid 32 covers the opening 30 to provide sanitary protection from any medical waste located within the container 10 because the container flaps 19 are at least partially covered by the medical waste bag 74 and, therefore are unable to cover the opening 30 without disturbing the placement of the medical waste bag 74 over the opening 30. Additionally, the lid 32 secures the medical waste bag 74 around the upper edge 24 of the container 10 by either frictionally engaging or snap-fitting with the container 10 to prevent the medical waste bag 74 from sliding off of the upper edge 24 into the container 10 when filling with medical waste.

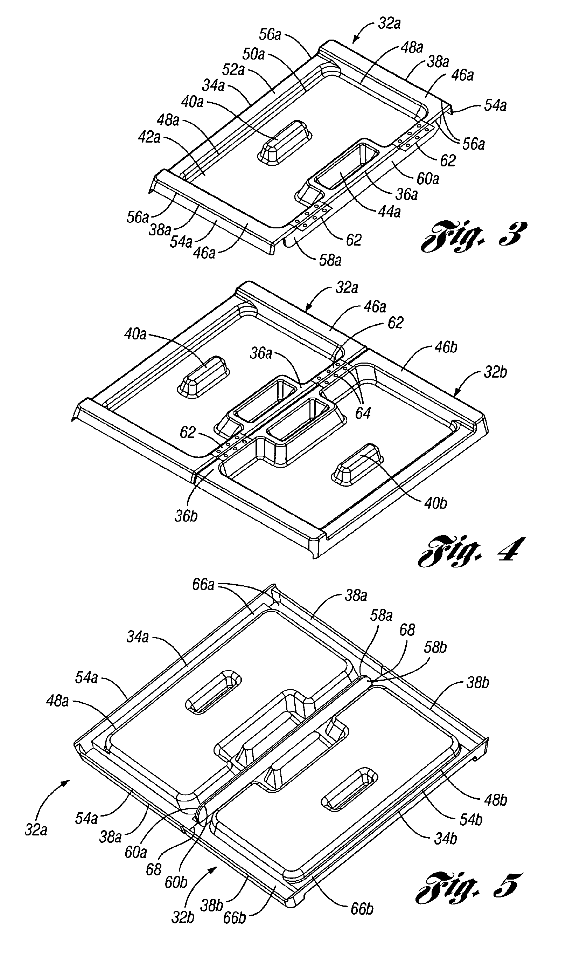

Figure 3 illustrates a top perspective view of a first portion 32a of the lid 32. The first portion 32a includes a front portion 34a, a rear portion 36a, and side portions 38a. At least one protruding handle 40a extends from a central portion of a lower surface 42a of the first portion 32a to provide a first grasping member. A recessed pocket 44a is formed in an upper surface 46a of the first portion 32a adjacent the rear portion 36a of the first portion 32a, but centre of the lid 32, to provide a second gripping member.

An inner wall 48a extends upwardly from a perimeter 50a of the lower surface 42a to an intermediate surface 52a along the front portion 34a and to the upper surface 46a along the side portions 38a and the rear portion 36a. An outer wall 54a extends downwardly from an outer edge 56a adjacent the side portions 38a and the front portion 34a. A first protrusion 58a extends downwardly from the outer edge 56a of the upper surface 46a adjacent the rear portion 36a. The first protrusion 58a includes an outwardly facing surface 60a. The first portion 32a and the second portion 32b are identical and can be produced from the same mold. The lid 32 may be thermoformed or injection molded. The lid 32 may also be reusable or recyclable.

Figure 4 illustrates a top perspective view of the first portion 32a and the second portion 32b pivotably connected by at least one hinge member 62. In this example, the at least one hinge member 62 is attached to the upper surfaces 46a 46b adjacent the rear portions 36a 36b by a plurality of fasteners 64, such as bolts, screws, or adhesive. Although the at least one hinge member 62 is shown attached to the upper surfaces 46a 46b, one of ordinary skill in the art would recognize that the at least one hinge member 62 could be mounted in a plurality of locations or molded into the lid 32 as a living hinge (by making the lid one-piece) without departing from the scope of the invention.

Figure 5 illustrates a bottom perspective view of the first portion 32a pivotably attached to the second portion 32b. The outwardly facing surface 60a of the first protrusion 58a is in contact with the outwardly facing surface 60b of the second protrusion 58b to prevent the first portion 32a from rotating relative to the second portion 32b past a certain threshold angle. Generally, the threshold angle is approximately 180 degrees.

A channel 66a, 66b is formed between the inner wall 48a, 48b and the outer wall 54a, 54b. The first and second protrusion 58a, 58b are spaced from the outer wall 54a, 54b to create a channel opening 68 to allow the upper hinge portion 26 of the container 10 to mate with the channel 66a, 66b. The channel 66a, 66b is deeper along the side portions 38a, 38b to accommodate the greater height of the upper hinged portion 26 on the side walls 14 and shallower along the front portion 34a, 34b to mate with the generally flat upper edge 28 of the front wall 16 and the rear wall 18. However, the depth of the channel 66a, 66b may vary to accommodate different containers 10. The inner wall 48a, 48b and the outer wall 54a, 54b along the front portion 34a, 34b of the lid 32 may engage the front wall 16 or the rear wall 18 to prevent the lid 32 from sliding on the container 10 when opening either the first portion 32a or the second portion 32b. The channels 66a, 66b, or portions thereof, may be slightly narrower than the upper edges of the front wall 16, rear wall 18 or side walls 14, such that the walls 48a, 48b and 54a, 54b are deformed slightly when installed to further retain the lid 32 on the container 10. Alternatively, or in addition, a snap-fit connection between the lid 32 and container 10 could be provided.

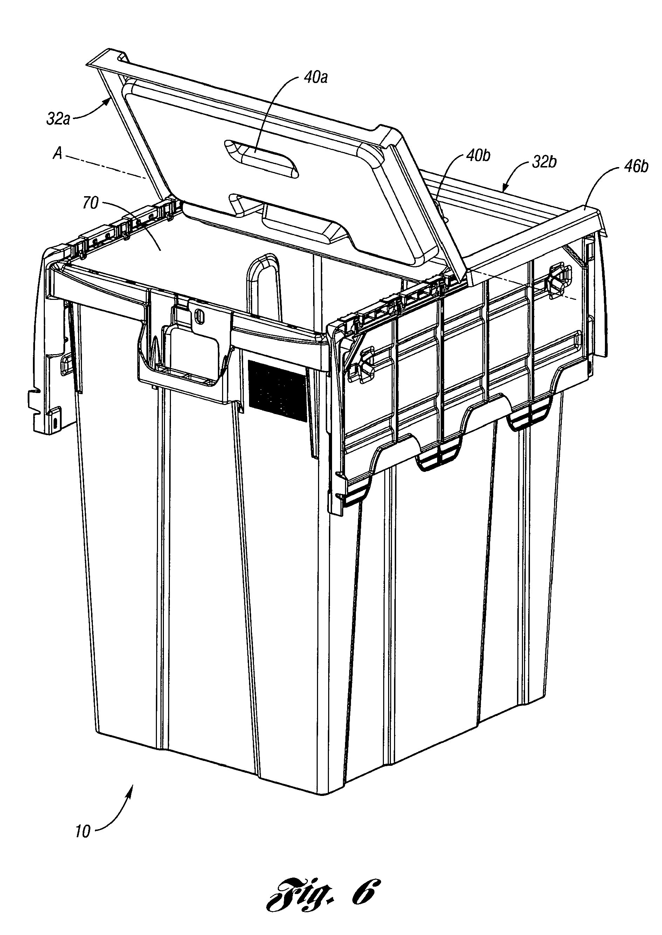

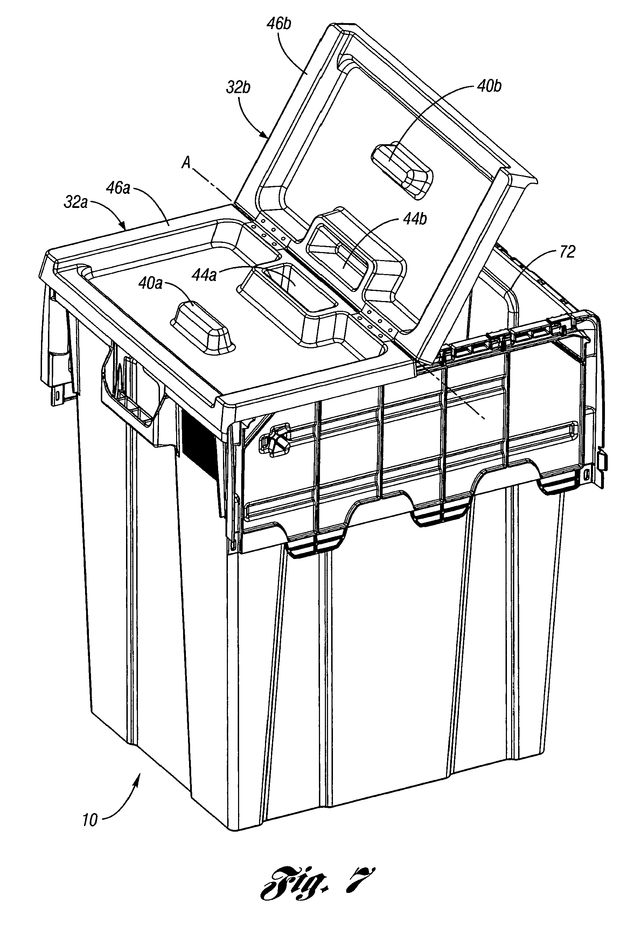

A first side 70 of the container 10 is accessed by engaging the protruding handle 40a on the first portion 32a and rotating the first portion 32a about axis of rotation A as shown in Figures 6. Similarly, a second side 72 of the container 10 is accessed by engaging the protruding handle 40b on the second portion 32b and rotating the second portion 32b about axis of rotation A as shown in Figures 7. The upper surface 46a of the first portion 32a may contact the upper surface 46b of the second portion 32b when accessing the first side 70 or the second side 72.

Alternatively, access to the entire opening 30 of the container 10 is obtained by simultaneously grasping with one hand the pockets 44a 44b on the lid 32 and applying a vertical lifting force to remove the lid 32. The first outwardly facing surface 60a on the first protrusion 58a engages the second outwardly facing surface 60b on the second protrusion 58b to maintain the lid 32 in a substantially flat orientation while allowing the pockets 44a 44b to be engaged by a single hand for removal. Note that in Figures 6 and 7, it is expected that the medical waste bag 74 would be used, but is not shown for clarity..

In accordance with the provisions of the patent statutes and jurisprudence, exemplary configurations described above are considered to represent a preferred embodiment of the invention. However, it should be noted that the invention can be practiced otherwise than as specifically illustrated and described without departing from its spirit or scope.

## Claims

1. A lid (32) for a waste container (10) comprising:
a first portion (32a) pivotably connected to a second portion (32b);
the first portion (32a) including a first handle (40a,44a) and the second portion (32b) including a second handle (40b,44b); and
a first protrusion (58a) extending from the first portion (32a) and a second protrusion (58b) extending from the second portion (32b) wherein the first protrusion (58a) engages the second protrusion (58b) to prevent the first portion (32a) from rotating relative to the second portion (32b) past a threshold angle.

2. The lid (32) of claim 1, wherein the first protrusion (58a) extends from a first edge (56a) on the first portion (32a) and the second protrusion (58b) extends from a second edge (56b) on the second portion (32b), the first portion (32a) connected by a hinge (62) to the second portion (32b) along the first edge (56a) and the second edge (56b).

3. The lid (32) of claim 1 or 2, wherein the first protrusion (58a) and the second protrusion (58b) are substantially perpendicular to an upper surface (46a,46b) of the lid (32).

4. The lid (32) of any preceding claim, wherein the first handle (44a) is located adjacent the second handle (44b).

5. The lid (32) of any of claims 1 to 3, wherein the first handle (40a) and the second handle (40b) are located in a central region of the first portion (32a) and the second portion (32b).

6. The lid (32) of any preceding claim, wherein a channel (66a,66b) extends about a perimeter of an underside of the lid (32).

7. The lid (32) of claim 6, wherein the channel (66a,66b) includes a first pair of opposite channels (34a,34b) at a first height and a second pair of opposite channels (38a,38b) are at a second height that is different from the first height.

8. The lid (32) of any preceding claim, wherein the threshold angle is approximately 180 degrees.

9. The lid (32) of any preceding claim, wherein the first portion (32a) is identical to the second portion (32b).

10. A container (10) and lid (32) assembly comprising:
a container (10) having a plurality of walls (12,14,16,18) defining an interior, the plurality of walls (12,14,16,18) having an upper perimeter (24);
a lid (32) including a first portion (32a) having a first protrusion (58a) and a second portion (32b) having a second protrusion (58b);
a channel (66a,66b) formed around a perimeter of the lid (32); and
the upper perimeter (24) of the container accepted within the channel (66a,66b) on the lid (32).

11. The container (10) and lid (32) assembly of claim 10, wherein a first pair of opposite channels (34a,34b) are at a first height and a second pair of opposite channels (38a,38b) are at a second height that is different from the first height.

12. The container (10) and lid (32) assembly of claim 11, wherein the second pair of channels (38a,38b) accepts an upper hinged portion (26) of the container (10).

13. The container (10) and lid (32) assembly of claim 11 or 12, wherein the first pair of opposite channels (34a,34b) prevents the lid (32) from sliding on the container (10) when opening the first portion (32a) or the second portion (32b).

14. A method of removing a lid (32) from a container (10) including:
a) engaging a first handle (44a) on a first portion (32a) and a second handle (44b) on a second portion (32a) of the lid (32);
b) applying a lifting force to the first handle (44a) and the second handle (44b) to remove the lid (32) from the container (10); and
c) engaging a first protrusion (58a) on the first portion (32a) with a second protrusion (58b) on the second portion (32b) of the lid (32) to prevent the first portion (32a) from rotating relative to the second portion (32b) past a threshold angle.

15. The method of claim 14, wherein the first handle (44a) is located adjacent the second handle (44b).
